(19) Europäisches Patentamt / European Patent Office / Office européen des brevets

(11) **EP 3 614 163 B1**

(12) **EUROPEAN PATENT SPECIFICATION**

(45) Date of publication and mention
of the grant of the patent:
**16.04.2025 Bulletin 2025/16**

(21) Application number: **18189964.2**

(22) Date of filing: **21.08.2018**

(51) International Patent Classification (IPC):
***G01R 33/389*** (2006.01)     ***G01R 33/3815*** (2006.01)

(52) Cooperative Patent Classification (CPC):
**G01R 33/389;** G01R 33/3815

(54) **METHOD OF OPERATING AN MRI APPARATUS**

VERFAHREN ZUM BETRIEB EINER MRT-VORRICHTUNG

PROCÉDÉ DE FONCTIONNEMENT D'UN APPAREIL D'IRM

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**26.02.2020 Bulletin 2020/09**

(73) Proprietor: **Siemens Healthineers AG
91301 Forchheim (DE)**

(72) Inventors:
• **BIBER, Stephan**
**91056 Erlangen (DE)**
• **POTTHAST, Andreas**
**91052 Erlangen (DE)**
• **VESTER, Markus**
**90471 Nürnberg (DE)**
• **NISTLER, Jürgen**
**91056 Erlangen (DE)**
• **DE OLIVEIRA, Andre**
**91080 Uttenreuth (DE)**
• **MODEL, Volker**
**90766 Fürth (DE)**

(74) Representative: **Siemens Healthineers
Patent Attorneys
Postfach 22 16 34
80506 München (DE)**

(56) References cited:
CN-A- 104 224 179       JP-A- H0 690 918
JP-A- H11 164 820       JP-B2- 6 328 911
US-A1- 2002 171 520

**Description**

The invention describes a method of operating an MRI apparatus. The invention further describes an MRI apparatus.

Developments in the field of magnetic resonance imaging (MRI) systems have led to advances in low-field systems, which can be preferred on account of their smaller footprint. Low-field systems can be open-bore, can allow interventional procedures, and are less expensive. The term "low-field system" generally refers to a system that has a magnetic field strength of at most 1.0 Tesla. An MRI system with a magnetic field strength in excess of 1.0 Tesla is generally referred to as a "high-field system". The magnetic field strength of the low-field systems currently in development may be even lower than 1.0 Tesla, and may even be lower than 0.5 Tesla.

When an MRI system is first installed on site, a ramping procedure is carried out to set up the main magnetic field (also referred to as the static background field) in the main coil windings. After the initial installation ramping procedure, shim coils are used to perform any adjustments necessary to take into account the local environment. Usually, the target frequency (i.e. the centre frequency of the main magnet) is determined with the aid of a probe placed at a suitable position in the apparatus. To allow for the inevitable decay of the magnetic field arising from component aging, typically in the order of several hundred ppm per year, this target frequency generally exceeds the centre frequency of the body coil by an amount that is sufficient to ensure that the centre frequency of the main magnetic field remains above the body coil centre frequency for as long as possible.

In any superconducting MRI system, decay of the magnetic field is unavoidable due to the residual electrical resistance of the magnet. Field decay means that the centre frequency of the main magnetic field gradually drifts away from the initial setting. Ultimately, it becomes necessary to re-ramp the system. In a high-field system, the bandwidth of the body coil and radio-frequency system is so large (e.g. body coil bandwidth $\pm$ 100 kHz or more) that it can take several years before field decay is out of specification. Generally, another service procedure such as a cold head exchange must be scheduled sooner. To carry out such a service procedure, the magnet must in any case be ramped down and up, so an opportunity is given to re-calibrate the system.

Before ramping the magnet, a target frequency for the main magnetic field is identified. It is usual to set the highest possible target frequency and to use the shim coils for any fine-tuning of the main magnetic field. By setting the highest possible target frequency, the decay window (the time taken for the frequency to drift to the lower end of the allowed band) is made as long as possible. This approach is suitable for systems with a high bandwidth as explained above. However, the bandwidth of a low-field system is significantly narrower than that of a high-field system, being only in the order of 10 kHz - 25 kHz, so that prior art methods of setting the magnet target frequency are limited to a much shorter "decay window". Because the decay window is narrower, the magnet frequency of a low-field MRI system decays towards an out-of-spec level in a shorter space of time. This means that low-field MRI systems must generally be ramped more often.

The first superconducting low-field MRI systems were generally realised as vertical field systems, characterized by a relatively inefficient body coil. These early low-field vertical systems are known to be less reliable than a comparable horizontal field system (with a birdcage body coil), so that service checks must be scheduled relatively frequently. During these service procedures, the frequency of the main magnetic field is checked and the system is re-ramped if necessary. In a low-field system, it may be necessary to carry out an intermittent ramping procedure, for example in the event of an infrastructure problem such as a loss of power or problems with a cooling issues arrangement, etc.

Document JP H11 164820 A a superconducting coil consisting of a high temperature superconductor and a compensating coil is disclosed, which are placed in a cooling container in a closely connected state. The compensating coil is connected with a compensating power source. After magnetization of the superconducting coil, current is supplied from the compensation power source to the compensating coil to compensate magnetic field attenuation with time in the static magnetic field of the superconducting magnet.

From document CN 104224179 a magnetic field stabilizing method and device for a magnetic resonance imaging system are known. According to the magnetic field stabilizing method, a group of active compensating coils is arranged in a magnet, real-time magnetic field frequency drift amount is acquired, the current magnitudes and change rates of the active compensating coils are set, and the magnetic field change of a main magnet is compensated in real time by using a generated magnetic field, so that a total magnetic field is kept stable, and drift of the magnetic field is eliminated.

Document JP H0 690918 A discloses a method to prevent the generation of positional deviation of a slice by deriving the shift from a reference frequency, that is, magnetic field correction data in accordance with the fluctuation of magnetic field intensity, comparing it with the allowable range of the magnetic field correction data inputted by an operator, and displaying an alarm message.

From document US 2002/0171520 A1 a superconducting magnet circuit is known which includes, besides a closed circuit formed of a plurality of unit superconducting coils connected in series and a first persistent-current switch, a second persistent-current switch which forms a closed circuit by connecting the ends of an arbitrary unit superconducting coil, and is therefore made up of the two closed circuits, whereby their mutual induction suppresses the decay of the magnet apparatus' central magnetic field to provide extreme stability.

Document JP 6 328911 B2 discloses a magnetic resonance imaging apparatus that collects maintenance data including the resonance frequency of a slice including the center of a magnetic field at a predetermined timing. A line scan execution unit for acquiring an echo signal of one line by executing an asymmetric spin echo method for one line along a predetermined direction including the magnetic field center using the resonance frequency of the slice and an echo signal of one line is provided. An anomaly degree determining unit determines an anomaly degree of the homogeneity of the magnetic field.

The object of the invention is to provide a way of monitoring the decay of the main magnetic field of an MRI apparatus. This object is achieved by the method of claim 1 of operating an MRI apparatus, and by the MRI apparatus of claim 8.

The method of operating an MRI apparatus comprises the steps of identifying a centre frequency during successive imaging procedures (i.e. during patient scans) and recording each centre frequency; and analysing the centre frequencies to identify a number of decay-related characteristics of the main magnetic field (usually referred to as the B0 field).

The MRI apparatus comprises an ammeter shunt arranged to measure magnet current while ramping, so that the magnet frequency can be estimated. When the magnet frequency has been observed to reach the pre-defined target frequency, it may be assumed that the magnetic field is satisfactory and the ramp procedure can be concluded. An ammeter shunt is an economical but low-accuracy component, and tends to become less accurate as it ages. Therefore, the MRI apparatus comprises a shunt monitoring module realised to derive an aging characteristic of the current sensor on the basis of patient scan centre frequencies. A current measurement is recorded at the end of a ramp-up sequence, e.g. when the magnet frequency has reached a target frequency. A calibration factor for the current sensor is derived from a relationship between the shunt current measurement and a subsequent centre frequency. The calibration factor may be expressed as a simple ratio, e.g. shunt current divided by centre frequency.

The shunt current measurement and a patient scan centre frequency measurement are temporally close. Since the shunt current measurement can only be made during a ramp-up procedure, the centre frequency measurement is made during a patient scan following the ramp-up. The calibration factor is used to correct the current sensor readings during a subsequent ramp-up sequence and can significantly improve the accuracy of the ramp-up sequence, thereby extending the magnet field decay window. It can also be used to correct for long-term aging effects of the shunt. The calibration factor is used by the algorithms that deduce the magnet frequency from the current sensor measurements. In this way, the MRI apparatus recalibrates this functionality in a straightforward manner, and prior to a next scheduled ramp-up procedure.

An advantage of the invention is that the main magnetic field can be monitored during normal operation of the MRI apparats, without the need for any dedicated measuring devices or additional components. The invention is based on the insight that the signal frequency detected at the receiver coil is related to the main magnetic field by the gyromagnetic ratio, and that the signal detected by the receiver coil can be used to deduce information relating to the temporal development of the magnetic field during successive scans. The field monitoring of the proposed method is advantageous especially in low-field systems, in which the magnet field decay must be remedied more frequently than an high-field systems. Very little additional effort is required to implement the inventive method, and essentially all steps can be carried out by suitable algorithms that process signals which are observed in any case during normal operation of the MRI apparatus.

An advantage of the invention is that a decay-related characteristic of the main magnetic field is derived entirely from measurements collected during patient scans, i.e. using measurements that are made in any case. No dedicated test measurements need be made. The measurements can be collected while adjusting the frequency of the receive system at the beginning of a patient scan, for example. Alternatively or in addition, measurements can be collected at a point during the actual imaging sequence. In either case, the signal for which the centre frequency is determined originates from the patient.

The MRI apparatus comprises a centre frequency identifier module realised to identify a centre frequency during an imaging procedure; a storage module realised to record centre frequencies identified during successive imaging procedures; and an analysis module realised to identify a number of decay-related characteristics of the main magnetic field from analysis of the recorded centre frequencies.

The units or modules of the MRI apparatus mentioned above, in particular the frequency determination unit and the selection unit, can advantageously be completely or partially realised as software modules running on a processor of a control unit of an MRI apparatus. A realisation largely in the form of software modules can have the advantage that applications already installed on an existing MRI system can be updated, with relatively little effort, to carry out the method steps of the present application. The object of the invention is also achieved by a computer program product with a computer program that is directly loadable into the memory of a control unit of an MRI apparatus, and which comprises program units to perform the steps of the inventive method when the program is executed by the control unit. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read from a processor unit of an MRI apparatus. A processor unit can comprise one or more microprocessors or their equivalents.

Particularly advantageous embodiments and features of the invention are given by the dependent claims, as revealed in the following description. Features of different claim categories may be combined as appropriate to give further embodiments not described herein.

The inventive method can be applied in any appropriate MRI apparatus that has a superconducting magnet. However, a low-field MRI apparatus with a superconducting magnet benefits to a greater extent from the inventive method.

In a particularly preferred embodiment of the invention, the field strength of the magnet is at most 1.0 T, more preferably at most 0.3 T. As indicated above, the RF bandwidth of a mid- or high-field MRI apparatus is generally very large, but the bandwidth of a low-field apparatus is significantly narrower and may be as small as 10 kHz, for example. In a further preferred embodiment of the invention, the MRI apparatus has a body coil with a RF bandwidth that does not exceed 50 kHz.

A centre frequency is determined during an imaging procedure from signals received by the body coil or the local coils. These signals are essentially the signals originating from the patient during a clinical imaging sequence.

As indicated above, the main magnetic field will inevitably decay over time. While a system can be manufactured to a high degree of accuracy and even though the magnet characteristics can be very precisely quantified, it is not feasible to predict exactly how a magnetic field will behave over several months or even years. However, it would be desirable for the customer (e.g. the radiology department of a clinic) to be informed of the status of the magnet at any time, so that routine maintenance events can be scheduled in a timely manner and so that down-time can be avoided.

In the inventive method, the recorded centre frequencies can be stored in a memory module and/or can be forwarded to a remote service database for remote monitoring of the system. An alert or notification can be issued to the customer or user when the frequency approaches or transcends the limits of a predefined tolerance window.

The recorded centre frequencies together with the maximum bandwidth and the decay-related characteristics make it possible to determine whether or not a centre frequency is still within a predefined window, and/or to predict when the centre frequency will leave the predefined window. A warning can be issued to the user or the customer service in good time, for example a few months or weeks ahead of the predicted time.

Therefore, in a particularly preferred embodiment of the invention, a decay-related characteristic comprises a rate of decay of the main magnetic field. The customer (or an online supervision system of a service IT infrastructure) can compare this information with the magnet specification to see whether the rate of decay is in spec (i.e. the magnetic field is decaying as predicted by the manufacturer) or whether the rate of decay deviates from the predicted rate. Particularly in the case of a higher decay rate, it is advantageous to be aware of this so that corrective measures can be planned accordingly. In a further preferred embodiment of the invention, a decay-related characteristic might be a decay level of the main magnetic field, for example the momentary percentage of the nominal field strength. The decay rate is effectively the rate of decay of the magnetic field, i.e. a first derivative ($dB/dt$). Another decay characteristic that may be of interest is the rate of change of the decay rate, i.e. the second derivative ($d^2B/dt^2$).

As explained above, a centre frequency is obtained when a patient scan is being carried out. Occasionally, it might happen that patient scan is not performed entirely correctly, for example when a metallic object is within the homogeneity volume, when the door of the radio-frequency cabin is inadvertently left open, etc., so that the measured centre frequency is not actually representative. Therefore, in a preferred embodiment of the invention, the method comprises a step of filtering an identified centre frequency prior to storing it. For example, a condition might be that an identified centre frequency may not differ by more than ±50 ppm from a preceding centre frequency. The filter is effectively a plausibility check that ensures that outliers are not included in the record of centre frequencies.

After subjecting an identified centre frequency to the plausibility check, it is recorded in a memory. A representative centre frequency value can be obtained by averaging the (filtered) centre frequencies measured during all patient scans during a specified time, for example during one day. The analysis unit can process a set of representative centre frequencies to detect any decay of the main magnetic field. Preferably, the analysis unit processes at least two representative centre frequencies, more preferably at least ten representative centre frequencies to derive a decay characteristic of the main magnetic field. For example, one representative centre frequency value can be obtained per day by averaging the centre frequencies measured during all patient scans made that day, and the decay-related characteristic can be computed at the end of the week. Similarly, one representative centre frequency value can be obtained from all patient scans made during one week, and the decay-related characteristic can be computed at the end of the month.

If a decay-related characteristic exceeds a predefined threshold, for example the magnet centre frequency has dropped below a certain level, or the decay rate has increased beyond a certain acceptable rate, the operator of the MRI apparatus is preferably informed. Therefore, in a particularly preferred embodiment of the invention, the method comprises a step of issuing an alert when a decay-related characteristic exceeds a predefined threshold.

The inventive method can be used to advantage to observe the magnet field strength over time, and to plan any corrective measures such as a partial ramp or automatic ramp. For example, analysis of the centre frequencies may indicate that the centre frequency of the magnetic field has decayed to a level that could be remedied by carrying out a partial ramp-up procedure which might be carried out in the evening (when the MRI apparatus is not in use). A partial or "top-up" ramp may take only a relatively short period of time compared to a complete ramp-up procedure which requires the magnet to be ramped down completely and then ramped up again to reach the desired magnet field strength.

During ramp-up, current is fed to the magnet until the magnet current or the corresponding frequency has reached a predefined target current or frequency. The target frequency can be determined in any suitable manner, for example by arranging a probe inside the body coil and monitoring the magnet current using a current sensor of the magnet power supply. The MRI apparatus can comprise a probe placement device for automatically placing the probe inside the body coil.

In a further preferred embodiment of the invention, any steps of analysing and evaluating the recorded centre frequencies are performed remotely, for example on an external server. A remote customer support service can analyse the collected information and schedule a ramp-up event for MRI apparatus, so that the customer does not need to be concerned about these tasks.

Other objects and features of the present invention will become apparent from the following detailed descriptions considered in conjunction with the accompanying drawings. It is to be understood, however, that the drawings are designed solely for the purposes of illustration and not as a definition of the limits of the invention.

Fig 1 shows a simplified block diagram of an MRI apparatus according to an embodiment of the invention;

Fig 2 shows a simplified circuit diagram of a superconductive low-field MRI apparatus according to an embodiment of the invention;

Fig 3 shows a graph of magnet field decay obtained using an embodiment of the inventive method;

Fig 4 shows a first graph of patient scan centre frequencies obtained using an embodiment of the inventive method.

In the diagrams, like numbers refer to like objects throughout. Objects in the diagrams are not necessarily drawn to scale.

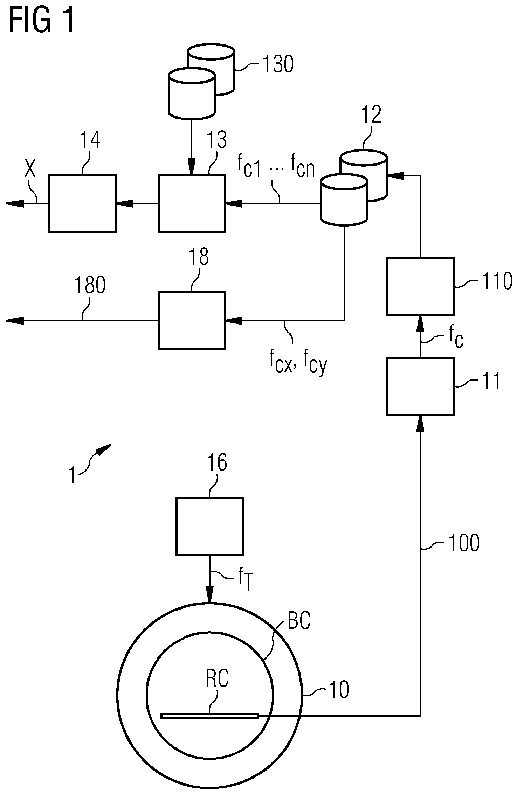

An MRI system comprises various modules and units, most of which will be known to the skilled person and need not be explained here. The apparatus comprises a main magnet that generates a very homogenous main magnetic field B0. Fig 1 shows a simplified block diagram of an MRI apparatus 1 indicating the main magnet 10, the body coil BC and a receiver coil RC (e.g. a flat receive-only spine coil in this example). The usual arrangement of additional coils such as shim coils, local coil, pickup coil and a number of gradient coils may be assumed to be present. During a patient scan, the body coil BC is excited to emit an RF signal, and the receiver coil RC detects a reflected RF signal 100. This signal 100 is processed by a module 11 which performs appropriate signal processing steps to identify the centre frequency $f_c$ of the received signal 100. To exclude any outliers, a filter is applied in block 110, which can compare the present centre frequency $f_c$ to the previous centre frequency. The present centre frequency $f_c$ is only approved if, for example, it does not differ from its predecessor by more than $\pm 50$ ppm. If approved, the present centre frequency $f_c$ is then stored in a memory module 12. These steps are repeated for successive patient scans, so that ultimately a collection of centre frequencies accumulates in the memory module 12.

The frequency of a received RC signal is related to the magnetic field strength by the relationship

$$ f = \frac{\gamma}{2\pi} B0 $$

where y is the gyromagnetic ratio. An analysis module processes a plurality of the successively collected centre frequencies $f_{c1}$, ..., $f_{c1}$ to identify a trend. For example, a gradual shift in frequency indicates a gradual decay of the main magnetic field. The analysis module can compare an identified decay trend with an expected decay trend known from the magnet specification, stored for example in a memory module 130. A decision module 14 can determined whether action needs to be taken on the basis of the information provided by the analysis unit 13. For example, the decision module may issue an alert X if a field strength decay rate is observed to be faster than an expected or specified decay rate.

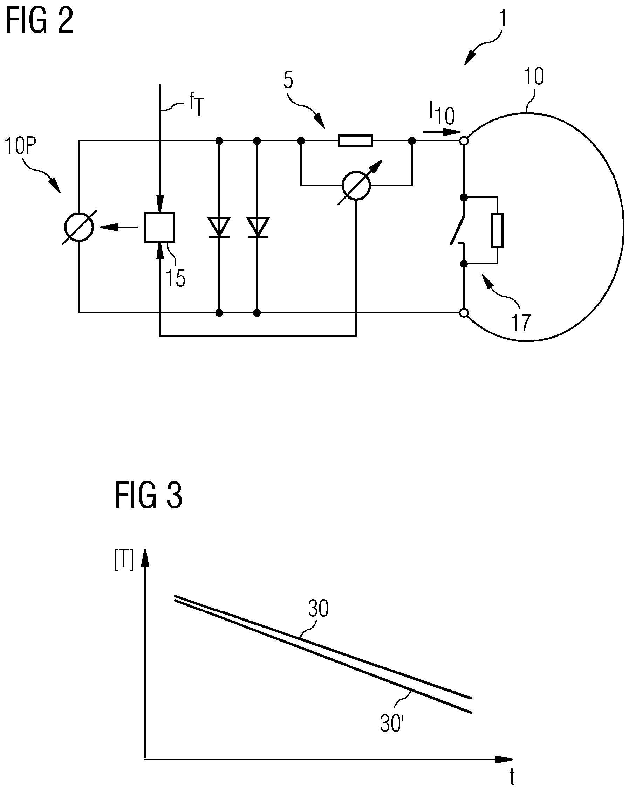

A ramp control module 16 is provided to initiate a ramping procedure at a suitable time, in order to ramp the main magnet 10 to a target frequency $f_T$. Fig 2 shows a greatly simplified circuit diagram of such a superconductive low-field MRI apparatus 1. An MPSU 10P is used to supply current $I_{10}$ to the magnet 10 during a ramp-up procedure, when the magnet 10 is ramped to the previously determined target frequency. A switch assembly 17 comprising a superconducting switch in parallel with a bypass resistor is shown connected across the main magnet coil 10. The switch is closed during the ramp-up procedure so that a small amount of current passes through the bypass resistor. An ammeter shunt S is used to measure the magnet current $I_{10}$ during ramping so that a power supply controller 15 can estimate the magnet frequency and compare it to the target frequency $f_T$ so that the ramp-up procedure can be halted when the target frequency $f_T$ has been attained. At this point, the switch 17 is opened again.

Returning to Fig 2, a current measurement is recorded at the end of a ramp-up sequence, e.g. when the magnet frequency has reached a target frequency. A calibration factor for the current sensor is derived from a relationship between the current measurement and a subsequent centre frequency. A calibration factor C may be expressed as a simple ratio of shunt current to centre frequency, e.g.

$$C = \frac{I_{10}}{f_c}$$

The shunt current measurement and a patient scan centre frequency measurement are preferably temporally close. Since the shunt current measurement can only be made during a ramp-up procedure, the centre frequency measurement is preferably made during the patient scan following the ramp-up.

The calibration factor C is used in a subsequent ramp-up procedure to correct for aging effects of the shunt S. The calibration factor C can be forwarded to the controller 15 of the MPSU 10P, for example, so that subsequent current readings can be corrected and the ramp-up procedure can be performed to a higher degree of accuracy.

The units and modules described above can be realized as part of a central control system of the MRI apparatus 1.

Fig 3 is a graph of magnet field decay showing magnet field strength (in Tesla) against time (in a timescale of months or even years). The diagram shows an expected decay profile 30, and a deduced decay profile 30' that is established by analysing a series of patient scan centre frequencies. In this exemplary case, the decay profile 31 shows a faster rate of decay than the expected decay profile 30, and may result in a decision to carry out a re-ramp event sooner than originally scheduled.

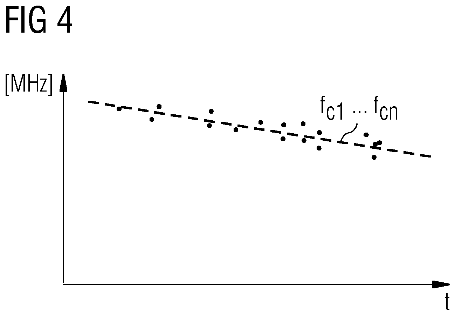

Fig 4 indicates how a collection of patient scan centre frequencies might be used to establish a magnet field decay characteristic. Here, the identified centre frequencies $f_{c1}$, ..., $f_{cn}$ are indicated by dots. The graph shows frequency on the X-axis (in MHz) against scan count along the Y-axis. The diagram shows a gradual decrease in the centre frequencies $f_{c1}$, ..., $f_{cn}$ of the detected RC signal. Using an appropriate interpolation or best-fit technique, the frequencies can be translated into a decay profile 31 of the main magnetic field as shown in Fig 3.

Although the present invention has been disclosed in the form of preferred embodiments and variations thereon, it will be understood that numerous additional modifications and variations could be made thereto without departing from the scope of the invention, provided that the resulting subject-matter falls within the scope of the invention as defined by the claims.

For the sake of clarity, it is to be understood that the use of "a" or "an" throughout this application does not exclude a plurality, and "comprising" does not exclude other steps or elements. The mention of a "unit" or a "module" does not preclude the use of more than one unit or module.

**Claims**

1. A method of operating an MRI apparatus (1), wherein the MRI apparatus comprises

   - a superconducting magnet (10),
   - a ramp control module (16) configured to initiate a ramping procedure at a suitable time to ramp the magnet (10) to a target frequency fT, and configured to feed, during a ramp-up procedure, current to the magnet until the magnet current or the corresponding magnet centre frequency has reached a predefined target current or frequency,
   - an ammeter shunt (5) arranged to measure the magnet current during a ramping procedure, so that the magnet frequency can be estimated and
   - a shunt monitoring module (14) configured to derive an aging characteristic of the ammeter shunt (S) on the basis of patient scan centre frequencies,
   which method comprises the steps of

   - identifying and recording centre frequencies ($f_{c1}$, ..., $f_{cn}$) during successive patient scans, wherein the centre frequencies are determined during the imaging procedure of a patient scan from signals received by a body coil or local coils of the MRI apparatus, and
   - analysing the centre frequencies ($f_{c1}$, ..., $f_{cn}$) to identify a number of decay-related characteristics of the main magnetic field of the MRI apparatus (1),
   - performing a ramp-up procedure, wherein during ramp-up, current is fed to the magnet (10) until the magnet current or the corresponding frequency has reached a predefined target current or frequency,

   **characterized by** the method further comprising the steps of recording a measurement of the ammeter shunt current at the end of the ramp-up procedure,

- determining an aging characteristic of the ammeter shunt(S) from the recorded magnet current measurement ($I_{10}$) and a centre frequency ($f_c$) identified in a patient scan following the ramp-up procedure,
- correcting the ammeter shunt current readings during a subsequent ramp-up procedure using the aging characteristic.

2. A method according to claim 1, wherein the step of analysing the centre frequencies ($f_{c1}$, ..., $f_{cn}$) to identify a decay-related characteristic comprises a step of averaging a plurality of centre frequencies ($f_{c1}$, ..., $f_{cn}$) measured during patient scans during a specified interval.

3. A method according to claim 1 or claim 2, wherein a decay-related characteristic comprises the rate of decay of the main magnetic field.

4. A method according to claim 3, wherein a decay-related characteristic comprises the rate of change of the decay rate of the main magnetic field.

5. A method according to any of the preceding claims, comprising a filtering step prior to storing the identified centre frequency ($f_{c1}$, ..., $f_{cn}$).

6. A method according to any of the preceding claims, comprising a step of issuing an alert (X) if a decay-related characteristic exceeds a predefined threshold.

7. A method according to any of the preceding claims, wherein the step of analysing the centre frequencies ($f_{c1}$, ..., $f_{cn}$) to identify a decay-related characteristic is performed at a remote location from the MRI apparatus (1).

8. An MRI apparatus (1) comprising

- a superconducting magnet (9),
- a ramp control module (16) configured to initiate a ramping procedure at a suitable time to ramp the magnet (10) to a target frequency fT, and configured to feed, during a ramp-up procedure, current to the magnet until the magnet current or the corresponding magnet centre frequency has reached a predefined target current or frequency,
- an ammeter shunt (S) arranged to measure magnet current ($I10$) during a ramping procedure, so that the magnet frequency can be estimated,
- a centre frequency identifier module (11) configured to identify a centre frequency ($f_{c1}$, ..., $f_{cn}$)

during a patient scan,
- a storage module (12) configured to record centre frequencies ($f_{c1}$, ..., $f_{cn}$) identified during successive imaging procedures,
- an analysis module (13) configured to identify a number of decay-related characteristics () of the main magnetic field from analysis of the recorded centre frequencies ($f_{c1}$, ..., $f_{cn}$),
**characterized by** the MRI apparatus further comprising
- a shunt monitoring module (14) configured to derive an aging characteristic of the ammeter shunt (S) on the basis of consecutive identified centre frequencies (fcx, fcy), each centre frequency being identified after a ramp-up procedure during an imaging procedure of a patient, and on the basis of corresponding current measurements (I10 ) obtained at the end of the respective ramp-up procedure,
- the MRI apparatus configured to correct the ammeter shunt current readings during a subsequent ramp-up procedure using the aging characteristic.

9. An MRI apparatus according to claim 8, wherein the field strength of the magnet (10) of the MRI apparatus (1) is at most 1.0 Tesla, more preferably at most 0.7 Tesla, most preferably at most 0.5 Tesla.

10. A computer program product comprising a computer program that is directly loadable into a memory of a control unit of an MRI apparatus (1) according to any of claims 8 to 9 and which comprises program elements for performing steps of the method according to any of claims 1 to 7 when the computer program is executed by the control unit.

11. A computer-readable medium on which is stored a computer program product according to claim 10.

**Patentansprüche**

1. Verfahren zum Betreiben einer MRT-Vorrichtung (1), wobei die MRT-Vorrichtung umfasst

- einen supraleitenden Magneten (10),
- ein Rampensteuermodul (16), das dazu ausgelegt ist, einen Rampenvorgang zu einem geeigneten Zeitpunkt zu initiieren, um den Magneten (10) auf eine Zielfrequenz fT hochzufahren, und dazu ausgelegt ist, während eines Hochfahrvorgangs Strom in den Magneten einzuspeisen, bis der Magnetstrom oder die entsprechende Magnetmittenfrequenz einen vordefinierten Zielstrom oder eine vordefinierte Zielfrequenz erreicht hat,
- einen Amperemeter-Nebenschluss (5), der angeordnet ist, um den Magnetstrom während eines Rampenvorgangs zu messen, so dass die Magnetfrequenz geschätzt werden kann, und
- ein Nebenschluss-Überwachungsmodul (14), das dazu ausgelegt ist, eine Alterungscharakteristik des Amperemeter-Nebenschlusses (S) basierend auf Mittenfrequenzen des Patientenscans abzuleiten,
wobei das Verfahren die Schritte umfasst:

- Identifizieren und Aufzeichnen von Mittenfrequenzen ($f_{c1}$, ..., $f_{cn}$) während aufeinander folgender Patientenscans, wobei die Mittenfrequenzen während des Bildgebungsvorgangs eines Patientenscans aus Signalen bestimmt werden, die von einer Körperspule oder lokalen Spulen der MRT-Vorrichtung empfangen werden, und
- Analysieren der Mittenfrequenzen ($f_{c1}$, ..., $f_{cn}$), um eine Reihe von abklingbedingten Eigenschaften des Hauptmagnetfeldes der MRT-Vorrichtung (1) zu identifizieren,
- Durchführen eines Hochfahrvorgangs, wobei während des Hochfahrens Strom in den Magneten (10) eingespeist wird, bis der Magnetstrom oder die entsprechende Frequenz einen vordefinierten Zielstrom oder eine vordefinierte Zielfrequenz erreicht hat,

**dadurch gekennzeichnet, dass** das Verfahren ferner die Schritte des Aufzeichnens einer Messung des Amperemeter-Nebenschlussstroms am Ende des Hochfahrvorgangs umfasst,

- Bestimmen einer Alterungscharakteristik des Amperemeter-Nebenschlusses (S) aus der aufgezeichneten Magnetstrommessung ($I_{10}$) und einer Mittenfrequenz ($f_c$), die in einem Patientenscan nach dem Hochfahrvorgang identifiziert wurde,
- Korrigieren der Ablesungen des Amperemeter-Nebenschlussstroms während eines nachfolgenden Hochfahrvorgangs unter Verwendung der Alterungscharakteristik.

2. Verfahren nach Anspruch 1, wobei der Schritt des Analysierens der Mittenfrequenzen ($f_{c1}$, ..., $f_{cn}$), um eine abklingbedingte Charakteristik zu identifizieren, einen Schritt des Mittelns einer Vielzahl von Mittenfrequenzen ($f_{c1}$, ..., $f_{cn}$) umfasst, die während Patientenscans während eines spezifizierten Intervalls gemessen werden.

3. Verfahren nach Anspruch 1 oder Anspruch 2, wobei eine abklingbedingte Charakteristik die Abklingrate des Hauptmagnetfeldes umfasst.

4. Verfahren nach Anspruch 3, wobei eine abklingbedingte Charakteristik die Änderungsrate der Abklingrate des Hauptmagnetfelds umfasst.

5. Verfahren nach einem der vorhergehenden Ansprüche, umfassend einen Filterschritt vor dem Speichern der identifizierten Mittenfrequenz ($f_{c1}$, ..., $f_{cn}$)

6. Verfahren nach einem der vorhergehenden Ansprüche, umfassend einen Schritt des Ausgebens einer Warnung (X), wenn eine abklingbedingte Charakteristik einen vordefinierten Schwellenwert überschreitet.

7. Verfahren nach einem der vorhergehenden Ansprüche, wobei der Schritt des Analysierens der Mittenfrequenzen ($f_{c1}$, ..., $f_{cn}$), um eine abklingbedingte Charakteristik zu identifizieren, an einem von der MRT-Vorrichtung (1) entfernten Ort durchgeführt wird.

8. MRT-Vorrichtung (1), umfassend:

- einen supraleitenden Magneten (9),
- ein Rampensteuermodul (16), das dazu ausgelegt ist, einen Rampenvorgang zu einem geeigneten Zeitpunkt zu initiieren, um den Magneten (10) auf eine Zielfrequenz fT hochzufahren, und dazu ausgelegt ist, während einer Hochfahrprozedur Strom in den Magneten einzuspeisen, bis der Magnetstrom oder die entsprechende Magnetmittenfrequenz einen vordefinierten Zielstrom oder eine vordefinierte Zielfrequenz erreicht hat,
- einen Amperemeter-Nebenschluss (S), der angeordnet ist, um den Magnetstrom ($I10$) während eines Rampenvorgangs zu messen, so dass die Magnetfrequenz geschätzt werden kann,
- ein Mittenfrequenz-Identifizierungsmodul (11), das dazu ausgelegt ist, während eines Patientenscans eine Mittenfrequenz ($f_{c1}$, ..., $f_{cn}$) zu identifizieren,
- ein Speichermodul (12), das dazu ausgelegt ist, Mittenfrequenzen ($f_{c1}$, ..., $f_{cn}$) aufzuzeichnen, die während aufeinander folgender Bildgebungsvorgänge identifiziert wurden,
- ein Analysemodul (13), das dazu ausgelegt ist, eine Anzahl von abklingbedingten Charakteristiken () des Hauptmagnetfelds aus der Analyse der aufgezeichneten Mittenfrequenzen ($f_{c1}$, ..., $f_{cn}$) zu identifizieren,

**dadurch gekennzeichnet, dass** die MRT-Vorrichtung ferner umfasst:

- ein Nebenschluss-Überwachungsmodul (14), das dazu ausgelegt ist, eine Alterungscharakteristik des Amperemeter-Nebenschlusses (S) basierend auf aufeinander folgenden identifizierten Mittenfrequenzen (fcx, fcy) abzuleiten, wobei jede Mittenfrequenz nach einem Hochfahrvorgang während eines Bildgebungsvorgangs eines Patienten und basierend auf entsprechenden Strommessungen (I10), die am Ende des jeweiligen Hochfahrvorgangs erhalten werden, identifiziert wird,
- die MRT-Vorrichtung dazu ausgelegt ist, die Ablesungen des Amperemeter-Nebenschlussstroms während eines nachfolgenden Hochfahrvorgangs unter Verwendung der Alterungscharakteristik zu korrigieren.

9. MRT-Vorrichtung nach Anspruch 8, wobei die Feldstärke des Magneten (10) der MRT-Vorrichtung (1) höchstens 1,0 Tesla, bevorzugter höchstens 0,7 Tesla, am meisten bevorzugt höchstens 0,5 Tesla beträgt.

10. Computerprogrammprodukt, umfassend ein Computerprogramm, das direkt in einen Speicher der Steuereinheit einer MRT-Vorrichtung (1) nach einem der Ansprüche 8 bis 9 ladbar ist, und das Programmelemente zum Durchführen von Schritten des Verfahrens nach einem der Ansprüche 1 bis 7 umfasst, wenn das Computerprogramm durch die Steuereinheit ausgeführt wird.

11. Computerlesbares Medium, auf dem ein Computerprogrammprodukt nach Anspruch 10 gespeichert ist.

**Revendications**

1. Un procédé pour faire fonctionner une installation (1) d'IRM, dans lequel l'installation d'IRM comprend

    - un aimant (10) supraconducteur,
    - un module (16) de commande de rampe configuré pour lancer une procédure de montée en rampe à un instant convenable pour amener en rampe l'aimant (10) à une fréquence fT cible, et configurer pour envoyer, pendant une opération de montée en rampe, du courant à l'aimant jusqu'à ce que le courant de l'aimant ou la fréquence centrale correspondante de l'aimant ait atteint un courant ou une fréquence cible défini à l'avance,
    - un shunt (5) d'ampèremètre monté pour mesurer le courant de l'aimant pendant une procédure de montée en rampe, de manière à pouvoir estimer la fréquence de l'aimant, et
    - un module (14) de contrôle de shunt configuré pour déduire une caractéristique de vieillissement du shunt (S) de l'ampèremètre sur la base de fréquences centrales de scan de patient,

lequel procédé comprend les stades de

    - identifier et enregistrer des fréquences ($f_{c1}$, ..., $f_{cn}$) centrales pendant des scans successifs de patient, dans lequel les fréquences centrales sont déterminées pendant la procédure d'imagerie d'un scan de patient à partir de signaux reçus par une bobine de corps ou des bobines locales de l'installation d'IRM, et
    - analyser les fréquences ($f_{c1}$, ..., $f_{cn}$) centrales pour identifier un nombre de caractéristiques en relation avec la décroissance du champ magnétique principal de l'installation (1) d'IRM,
    - effectuer une procédure de montée en rampe, dans lequel pendant une montée en rampe, du courant est envoyé à l'aimant (10) jusqu'à ce que le courant de l'aimant ou la fréquence correspondante ait atteint un courant et/ou une fréquence cible défini à l'avance,

**caractérisé en ce que** le procédé comprend en outre les stades d'enregistrement d'une mesure du courant de shunt de l'ampèremètre à la fin de la procédure de montée en rampe,

    - de détermination d'une caractéristique de vieillissement du shunt (S) de l'ampèremètre à partir de la mesure ($I_{10}$) de courant d'aimant enregistrée et d'une fréquence ($f_c$) centrale identifiée dans un scan de patient à la suite de la procédure de montée en rampe,
    - de correction des indications de courant de shunt d'ampèremètre pendant une procédure subséquente de montée en rampe en utilisant la caractéristique de vieillissement.

2. Un procédé suivant la revendication 1, dans lequel le stade d'analyse des fréquences ($f_{c1}$, ..., $f_{cn}$) centrales pour identifier une caractéristique en relation avec une décroissance comprend un stade dans lequel on fait une moyenne d'une pluralité de fréquences ($f_{c1}$, ..., $f_{cn}$) centrales mesurées pendant des scans de patient pendant un intervalle précisé.

3. Un procédé suivant la revendication 1 ou la revendication 2, dans lequel une caractéristique en relation avec une décroissance comprend la vitesse de décroissance du champ magnétique principal.

4. Un procédé suivant la revendication 3, dans lequel une caractéristique en relation avec une décroissance comprend la vitesse de variation de la vitesse de décroissance du champ magnétique principal.

5. Un procédé suivant l'une quelconque des revendications précédentes, comprenant un stade de filtrage avant de mettre en mémoire la fréquence ($f_{c1}$, ..., $f_{cn}$) centrale identifiée.

6. Un procédé suivant l'une quelconque des revendications précédentes, comprenant un stade d'émission d'une alerte (X) si une caractéristique en relation avec une décroissance dépasse un seuil défini à l'avance.

7. Un procédé suivant l'une quelconque des revendications précédentes, dans lequel le stade d'analyse des fréquences ($f_{c1}$, ..., $f_{cn}$) centrales pour identifier une caractéristique en relation avec une décroissance est effectué en un emplacement loin de l'installation (1) d'IRM.

8. Une installation (1) d'IRM comprenant

   - un aimant (9) supraconducteur,
   - un module (16) de commande de rampe configuré pour lancer une procédure de montée en rampe à un instant convenable pour amener en rampe l'aimant (10) à une fréquence fT cible, et configurer pour envoyer, pendant une opération de montée en rampe, du courant à l'aimant jusqu'à ce que le courant de l'aimant ou la fréquence centrale correspondante de l'aimant ait atteint un courant ou une fréquence cible défini à l'avance,
   - un shunt (S) d'ampèremètre monté pour mesurer le courant (I10) de l'aimant pendant une procédure de montée en rampe, de manière à pouvoir estimer la fréquence de l'aimant,
   - un module (11) d'identificateur d'une fréquence centrale configuré pour identifier une fréquence ($f_{c1}$, ..., $f_{cn}$) centrale pendant un scan de patient,
   - un module (12) de mémoire configuré pour enregistrer des fréquences ($f_{c1}$, ..., $f_{cn}$) centrales identifiées pendant des procédures d'imagerie successives,
   - un module (13) d'analyse configuré pour identifier un nombre de caractéristiques ( ) en relation avec une décroissance du champ magnétique principal, à partir d'une analyse des fréquences ($f_{c1}$, ... $f_{cn}$) centrales enregistrées,
   **caractérisée en ce que** l'installation d'IRM comprend en outre
   - un module (14) de contrôle de shunt configuré pour déduire une caractéristique de vieillissement du shunt (S) de l'ampèremètre sur la base de fréquences (fcx, fcy) centrales identifiées consécutives, chaque fréquence centrale étant identifiée après une procédure de montée en rampe pendant une procédure d'imagerie d'un patient et sur la base de mesures (I10) correspondantes de courant obtenues à la fin de la procédure respective de montée en rampe,
   - l'installation d'IRM étant configurée pour corriger des valeurs de courant de shunt d'ampèremètre pendant une procédure de montée en rampe subséquente en utilisant la caractéristique de vieillissement.

9. Une installation d'IRM suivant la revendication 8, dans laquelle l'intensité de champ de l'aimant (10) de l'installation (1) d'IRM est au plus de 1,0 tesla, d'une manière plus préférée d'au plus 0,7 tesla, d'une manière la plus préférée d'au plus 0,5 tesla.

10. Produit de programme d'ordinateur comprenant un programme d'ordinateur, qui peut être chargé directement dans la mémoire d'une unité de commande d'une installation (1) d'IRM suivant l'une quelconque des revendications 8 à 9 et qui comprend des éléments de programme pour effectuer les stades du procédé suivant l'une quelconque des revendications 1 à 7, lorsque le programme d'ordinateur est exécuté par l'unité de commande.

11. Support, déchiffrable par ordinateur, sur lequel est mis en mémoire un produit de programme d'ordinateur suivant la revendication 10.

# FIG 1

**FIG 2**

**FIG 3**

# FIG 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H11164820 A
- CN 104224179
- JP H0690918 A
- US 20020171520 A1
- JP 6328911 B